(19) Europäisches Patentamt
European Patent Office
Office européen des brevets

(11) Veröffentlichungsnummer: **0 018 515 B1**

(12) # EUROPÄISCHE PATENTSCHRIFT

(45) Veröffentlichungstag der Patentschrift: **27.10.82**

(51) Int. Cl.³: **C 07 J 9/00,** C 07 J 31/00, C 12 P 33/06

(21) Anmeldenummer: **80101893.8**

(22) Anmeldetag: **09.04.80**

(54) **Verfahren zur Herstellung von Chenodeoxycholsäure und Zwischenprodukte dazu.**

(30) Priorität: **12.04.79 US 29420**

(43) Veröffentlichungstag der Anmeldung: **12.11.80 Patentblatt 80/23**

(45) Bekanntmachung des Hinweises auf die Patenterteilung: **27.10.82 Patentblatt 82/43**

(84) Benannte Vertragsstaaten: **AT BE CH DE FR GB IT LI NL**

(56) Entgegenhaltungen:
**FR-A-2 342 338**
**US-A-3 891 681**

(73) Patentinhaber: **F. HOFFMANN-LA ROCHE & CO. Aktiengesellschaft, CH-4002 Basel (CH)**

(72) Erfinder: **Despreaux, Carl, 23 Daniel Drive, Cedar Grove, N.J. 07009 (US)**
Erfinder: **Narwid, Thomas Albert, 7 Brooklawn Drive, Pompton Plains, N.J. 07444 (US)**
Erfinder: **Palleroni, Norberto J., 47 White Oak Drive, North Caldwell, N.J. 07006 (US)**
Erfinder: **Uskokovic, Milan R., 253 Highland Avenue, Upper Montclair, N.J. 07043 (US)**

(74) Vertreter: **Lederer, Franz, Dr. et al, Patentanwälte Dr. Franz Lederer Reiner F. Meyer Lucile-Grahn-Strasse 22, D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 018 515 B1

BUNDESDRUCKEREI BERLIN

# Verfahren zur Herstellung von Chenodeoxycholsäure und Zwischenprodukte dazu

Die vorliegende Erfindung betrifft ein Verfahren zur Herstellung von Chenodeoxycholsäure der Formel

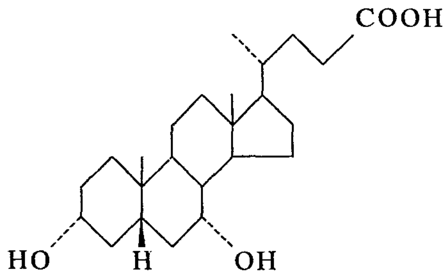

(I)

aus 3-Keto-bisnorcholenol (22-Hydroxy-23,24-bisnorchol-4-en-3-on), einer Verbindung der Formel

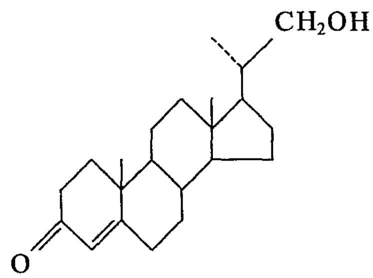

(II)

die leicht durch mikrobiologischen Abbau des im Handel erhältlichen β-Sitosterols nach bekannten Verfahren erhältlich ist.

In einer Beziehung betrifft die Erfindung ein Verfahren zur Herstellung von Chenodeoxycholsäure, welches dadurch gekennzeichnet ist, daß man (5β)-24-Norcholan-3α,7α-diol-23,23-dicarbonsäure thermisch decarboxyliert.

Ein weiterer Aspekt der Erfindung betrifft ein Verfahren zur Herstellung von (5β)-24-Norcholan-3α,7α-diol-23,23-dicarbonsäure aus 3-Keto-bis-norcholenol, welches Verfahren dadurch gekennzeichnet ist, daß man

A) 3-Keto-bisnorcholenol mittels eines Mikroorganismus der Gruppe Botryodiplodia theobromae IFO 6469, DSM 62-678; DSM 62-679; Lasiodiplodia theobromae ATCC 28 570; Botryosphaeria ribis ATCC 22 802, B. berengeriana ATCC 12 557, B. rhodina CBS 374.54, CBS 287.47 und CBS 306.58 oder einem Enzymextrakt davon zu 7α-Hydroxy-3-keto-bis-norcholenol hydrolysiert;

B) das 7α-Hydroxy-3-keto-bisnorcholenol in Gegenwart eines Palladiumkatalysators zu (5β)-7α,22-dihydroxy-23,24-bisnorcholan-3-on hydriert,

C) das (5β)-7α-22,Dihydroxy-23,24-bisnorcholan-3-on mit einem Methyl- oder p-Tolylsulfonylhalid zu einer Verbindung der Formel

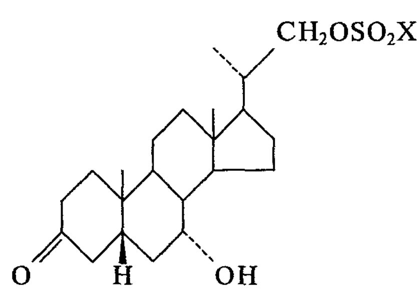

(III)

worin X Methyl oder p-Tolyl ist,

umsetzt und entweder

D) das Produkt von Stufe C) mit einem Natrium-di-$C_{1-3}$-alkyl-malonat zu einer Verbindung der Formel

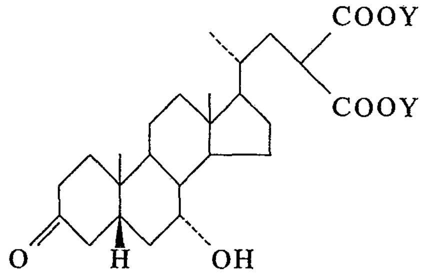

(IV)

worin Y $C_{1-3}$-Alkyl ist,
umsetzt;

E) das Produkt der Stufe D) mit einem chemischen Reduktionsmittel behandelt, um eine Verbindung der Formel

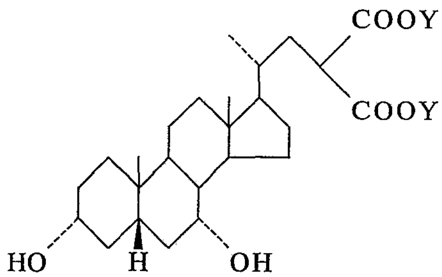

(V)

worin Y $C_{1-3}$-Alkyl ist,
zu erhalten; und

F) das Produkt der Stufe E) mit einer starken Base zu (5$\beta$)-24-Norcholan-3$\alpha$, 7$\alpha$-diol,23,23-dicarbonsäure verseift; oder

G) das Produkt der Stufe C) mit einem chemischen Reduktionsmittel behandelt, um eine Verbindung der Formel

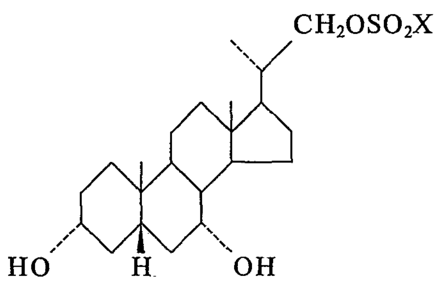

(VI)

worin X die obige Bedeutung hat,
zu erhalten;

H) das Produkt der Stufe G) mit einem mehr als zweifachen molaren Überschuss eines in der Steroidchemie zum Schutze von Hydroxygruppen üblichen Acylierungsmitels zu einer Verbindung der Formel

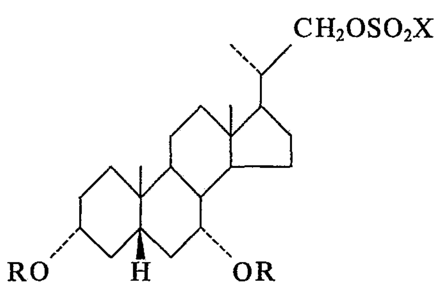

(VII)

worin X die obige Bedeutung hat und R Acyl ist,
umsetzt;

I) das Produkt der Stufe H) mit einem Natrium-di-$C_{1-3}$-alkylmalonat zu einer Verbindung der Formel

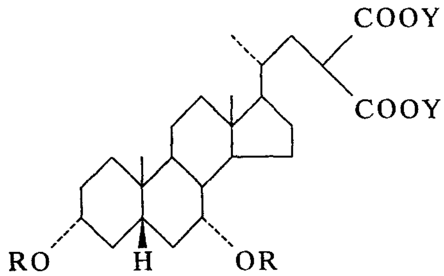

(VIII)

worin Y $C_{1-3}$-Alkyl ist und R die obige Bedeutung hat, umsetzt; und

J) das Produkt der Stufe I) mit einer starken Base zu (5β)-24-Norcholan-3α,7α-diol-23,23-dicarbonsäure verseift.

In der ersten Verfahrensstufe der vorliegenden Erfindung wird 3-Keto-bisnorcholenol in 7-Stellung mikrobiologisch hydroxyliert, wobei 7α-Hydroxy-3-keto-bisnorcholenol(7α,22-dihydroxy-23,24-bisnorchol-4-en-3-on) erhalten wird.

Unerwarteterweise wurde gefunden, daß von 152 Kulturen, die auf 7α-Hydroxylierung geprüft wurden und die 92 Spezies in 41 Genera repräsentierten, wovon vielen die Fähigkeit zur 7-Alphahydroxylierung der verschiedenen oben aufgezählten Verbindungen zugeschrieben worden war, nur 9 sehr nahe verwandte Kulturen, nämlich Botryodiplodia theobromae IFO 6469, ATCC 28 570, DSM 62-678, DSM 62-679; Botryosphaeria ribis ATCC 22 802, B. berengeriana ATCC 12 557, B. rhodina CBS 374.54, CBS 287.47 und CBS 306.58 in der Lage sind die gewünschte 7α-Hydroxylierung dieses Steroidsubstrats durchzuführen.

Die Mikroorganismen können in Form der Kulturlösung, des Mycels oder eines Enzymextraktes daraus angewendet werden. Die Kulturlösung kann durch Beimpfen eines geeigneten Mediums mit dem Organismus hergestellt werden. Das Kulturmedium kann Kohlenstoffquellen, Stickstoffquellen, anorganische Salze und andere für das Wachstum des Mikroorganismus geeignete Nährstoffe enthalten. Kohlenstoffquellen sind beispielsweise Glukose, Sucrose, Dextrin, Mannose, Stärke, Lactose oder Glycerin; Stickstoffquellen sind beispielsweise stickstoffhaltige Substanzen wie Peptone, Fleischextrakt, Hefeextrakt, Kornsteepliquor oder Casein oder stickstoffhaltige anorganische Verbindungen wie Nitrate oder anorganische Ammoniumsalze. Beispiele anorganischer Salze sind Phosphate und Mineralsalze wie Natrium, Kalium, Magnesium, Mangan, Eisen- und Kupfersalze.

Als Kultivierungsmethode können Submerskulturen, Schüttelkulturen oder stationäre Kulturen verwendet werden. Da aerobe Bedingungen für die erfindungsgemäß verwendeten Organismen erforderlich sind, ist es vorzuziehen, unter Bedingungen zu kultivieren, die die Belüftung fördern.

Es ist bei der erfindungsgemäßen Hydroxylierung auch möglich, ein von der Kulturlösung des Mikroorganismus isoliertes Mycel oder einen rohen Enzymextrakt der aus der Kulturlösung oder dem Mycel in an sich bekannter Weise hergestellt worden ist, zu verwenden und unter geeigneten Bedingungen mit dem Substrat in Kontakt zu bringen. In einem solchen Fall kann die 7α-Hydroxylierung zweckmäßig in wäßriger Lösung, wie in einer Pufferlösung, in physiologischer Kochsalzlösung, in frischem Nährmedium oder in Wasser durchgeführt werden.

Das Substrat kann in Pulverform oder als Lösung in einem hydrophilen Lösungsmittel wie Aceton, Dimethylsulfoxid, Methanol, Äthanol, Äthylenglykol, Propylenglykol oder Dioxan zugesetzt werden. Einer Substratsuspension in Wasser kann ein oberflächenaktives oder Dispersionsmittel zugesetzt werden. Weiterhin kann das Substrat mittels Ultraschall feinsuspendiert werden.

Das Fermentierungsverfahren verwendet konventionelle Arbeitstechniken. So kann der Mikroorganismus in einer Edaminbrühe (die gleiche wie das Fermentationsmedium, siehe unten) während 18 bis 72 Stunden bei etwa 15–35°C angezüchtet werden. Die Fermentation wird eingeleitet, indem man ein konventionelles Fermentationsmedium mit 1 bis 10 Gewichtsprozent Biomasse beimpft. Die Fermentationsbedingungen können die gleichen sein wie bei der Anzucht des Inoculums. Nach einer Inkubationsperiode von 18 bis 96 Stunden wird das Substrat entweder als Lösung, vorzugsweise in absolutem Äthanol, oder als unter Beschallung hergestellte Lösung in 0,1% Tween 80® (Polyoxyäthylen-sorbitan-monoleat) zugegeben. Die Fermentation kann bis zu 120 Stunden nach Zugabe des Substrats fortgesetzt werden. Ein geeignetes Fermentationsmedium wird beispielsweise durch Mischen folgender Bestandteile erhalten: 20 g Edamin® (ein durch enzymatischen Abbau von Lactalbumin hergestelltes Produkt der Sheffield Chemical Co.) 3 g Cornsteepliquor, 50 g Dextrose und destilliertes Wasser zu einem Liter. Das pH des Mediums wird auf etwa 4 bis 7, vorzugsweise auf pH 5, vor der Sterilisierung (die z. B. durch Autoklavieren durchgeführt werden kann) eingestellt.

Die Isolierung des 7α-Hydroxy-3-keto-bisnorcholenol aus dem Fermentationsmedium kann leicht nach an sich bekannten Verfahren durchgeführt werden. So kann die gesamte Kulturlösung mit einem

nicht mischbaren organischen Lösungsmittel, wie vorzugsweise Äthylacetat, extrahiert werden. Die Lösungsmittelfraktionen können dann mittels Gelchromatographie, z. B. Silicagel G-60, und anschließende Kristallisation gereinigt werden.

Es wurde weiter gefunden, daß eine Anzahl von Verfahren zur Optimierung der Ausbeute an Fermentationsprodukt angewandt werden kann. Beispiele dafür sind der Zusatz eines Chelatbildners, wie 2,2'-Dipyridyl in einer Endkonzentration von $0{,}5 \times 10^{-4}$ bis $0{,}75 \times 10^{-3}$ Mol, gleichzeitiger Zusatz von Glukose oder Sucrose mit dem Substrat in einer Endkonzentration von etwa 5%, Herabsetzung der Fermentationstemperatur auf etwa 24°C nach Zusatz des Substrats und Anwendung einer Substratkonzentration von 5% in 0,1% Tween 80®. Eine noch größere Ausbeutesteigerung ist möglich durch Zusatz von Adsorbentien zum Fermentationsmedium. Beispielsweise wird eine Verbesserung der Ausbeute erreicht, wenn polymere Harz-Adsorbentien wie Amberlit XAD7® (ein polymerer Acrylsäuremethylester der Rohm & Haas Co.) in einer Konzentration von 0,3 bis 0,6 Gewichtsprozent einem Fermentationsmedium zugesetzt wird in dem das Substrat in einer Konzentration von bis etwa 1 g pro Liter anwesend ist. Beste Ausbeuten wurden erreicht mit einer Konzentration von etwa 0,6 Gewichtsprozent an Adsorptionsmittel.

Das so hergestellte 7α-Hydroxy-3-keto-bisnorcholenol wird dann katalytisch zu (5β)-7α,22-dihydro-23,24-bisnorcholan-3-on hydriert.

Ein geeigneter Katalysator für diese Hydrierung ist Palladium, vorzugsweise auf einem festen Träger, insbesondere 5% Palladium auf Kohle. Die Reaktion wird zweckmäßig in einem nicht wäßrigen polaren Lösungsmittel wie Dimethylformamid und bei einer Temperatur von etwa 0–50°C, vorzugsweise bei Raumtemperatur und Normaldruck, ausgeführt. Das Reaktionsprodukt kann in der gleichen Weise wie oben bei der Fermentation beschrieben, d. h. durch Gelchromatographie und Kristallisation isoliert werden.

Das (5β)-7α,22-Dihydro-23,24-bisnorcholan-3-on wird dann mit einem p-Tolyl oder Methylsulfonylhalid zweckmäßig im Bereich von –78 bis 0°C, vorzugsweise bei –10°C, umgesetzt. Die Reaktion wird zweckmäßig in einem stickstoffhaltigen organischen Lösungsmittel, wie Pyridin, ausgeführt. Ein bevorzugtes Reagens für diese Umsetzung ist p-Toluolsulfochlorid. Das Reaktionsprodukt, eine Verbindung der Formel

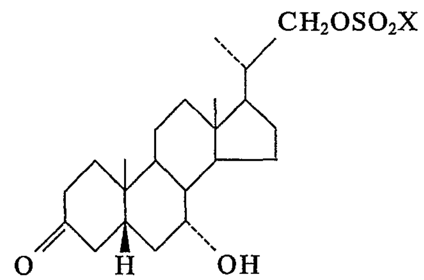

(III)

kann in der gleichen Weise wie für die obengenannten Verbindungen beschrieben isoliert werden.

Eine bevorzugte Verbindung III ist (5β)-7α-Hydroxy-22-([4-methylphenyl]sulfonyl-oxy)-23,24-bisnorcholan-3-on.

Im nächsten Schritt des erfindungsgemäßen Verfahrens wird die Verbindung der Formel III mit Natrium-di-$C_{1-3}$-alkylmalonat, vorzugsweise Dimethylmalonat, zweckmäßig in einem polaren nicht wäßrigen Lösungsmittel wie Dimethylformamid bei Temperaturen zwischen 0 und 100°C, vorzugsweise bei 50°C, unter Feuchtigkeitsausschluß in Inertgasatmosphäre, umgesetzt. Das Natrium-di-$C_{1-3}$-Alkylmalonat kann in situ durch Zusatz des Malonats zu einer Lösung von Natriumhydrid in Dimethylformamid und Rühren bei 40–50°C hergestellt werden. Die Isolierung des Verfahrensproduktes, einer Verbindung der Formel

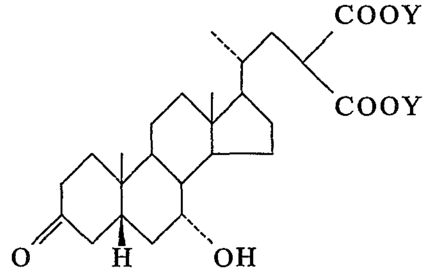

(IV)

kann in Analogie zu den oben beschriebenen Verfahren vorgenommen werden. Eine bevorzugte Verbindung der Formel IV ist (5β)-24-Norcholan-7 α-ol-3-on-23,23-dicarbonsäuredimethylester.

Die Verbindung der Formel IV wird dann zur 3-Hydroxyverbindung der Formel

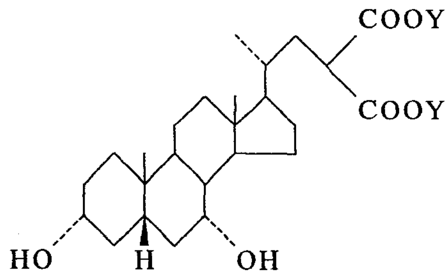

(V)

z. B. zu (5$\beta$)-24-Norcholan-3$\alpha$,7$\alpha$-diol-23,23-dicarbonsäuredimethylester reduziert.

Die Reduktion wird zweckmäßig mit einem konventionellen chemischen Reduktionsmittel wie Natriumborhydrid, in einem wäßrigen $C_{1-3}$-Alkanol als Lösungsmittel, wie z. B. 95%igem Äthanol, bei 0–50°C, vorzugsweise bei Raumtemperatur unter Inertgasatmosphäre, ausgeführt. Das Reaktionsprodukt kann aus dem Reaktionsgemisch durch Ansäuern und Extraktion mit einem halogenierten Kohlenwasserstoff, wie Dichloräthan, isoliert werden. Entfernung des Lösungsmittels liefert das Produkt in roher Form in der es ohne weitere Reinigung weiter verwendet werden kann.

Das Diol der Formel V wird dann durch Rückflußerhitzen in Gegenwart einer starken Base, z. B. Bariumhydroxid, verseift und liefert nach Aufarbeitung und Ansäuern die (5$\beta$)-24-Norcholan-3$\alpha$,7$\alpha$-diol-23,23-dicarbonsäure.

Die letzte Stufe des erfindungsgemäßen Verfahrens besteht darin, daß die Dicarbonsäure thermisch decarboxyliert wird, z. B. durch Erhitzen auf 190–205°C unter Inertgasatmosphäre, wobei Chenodeoxycholsäure erhalten wird.

In einer alternativen Ausführungsform des erfindungsgemäßen Verfahrens kann eine Verbindung der Formel III mit einem chemischen Reduktionsmittel, wie Lithiumaluminiumalkoxyhydrid, vorzugsweise Lithiumaluminium-tri-t-butoxyhydrid, bei etwa –78°C bis Raumtemperatur, vorzugsweise bei etwa –10°C, in einem inerten organischen Lösungsmittel, wie einem cyclischen Äther, vorzugsweise Tetrahydrofuran, unter Inertgasatmosphäre zu einem Diol der Formel

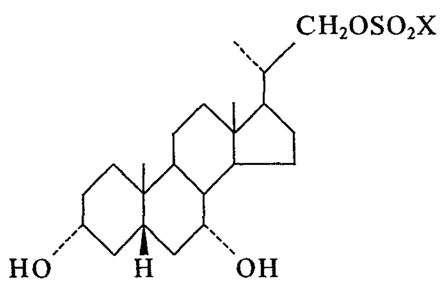

(VI)

reduziert werden.

Eine bevorzugte Verbindung der Formel VI ist 2,2-([4-Methylphenylsulfonyl]oxy)-23,24-bisnorcholan-3$\alpha$,7$\alpha$-diol.

Die Verbindung der Formel VI wird dann mit einem mehr als zweifachen molaren Überschuß eines Acylierungsmittels, das zum Schutz von Hydroxygruppen in der Steroidchemie geläufig ist, umgesetzt und liefert die entsprechende Diacylverbindung. Geeignete Acylierungsmittel sind $C_{2-6}$-Niederalkancarbonsäureanhydride, vorzugsweise Acetanhydrid. Die Acylierung wird zweckmäßig in einem geeigneten organischen Lösungsmittel, wie Pyridin, in Gegenwart einer Aminbase, wie 4-Dimethylaminopyridin, ausgeführt wobei man das Diacylderivat der Formel

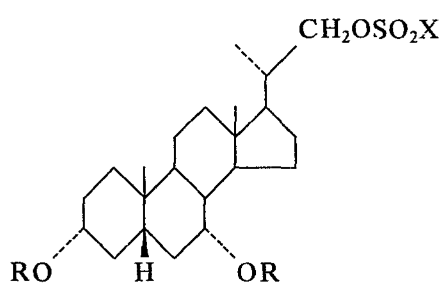

(VII)

z. B. 22-([4-Methylphenyl)-sulfonyl]oxy)-23,24-bisnorcholan-3$\alpha$,7$\alpha$-diol-3,7-diacetat erhält.

Die Diacylverbindung der Formel VII wird dann mit einem Natrium-di-$C_{1-3}$-alkylmalonat, vorzugsweise Diäthylmalonat, in Analogie zu der oben beschriebenen Umsetzung der Verbindung der

Formel III zu einer Verbindung IV behandelt, wobei man eine Verbindung der Formel

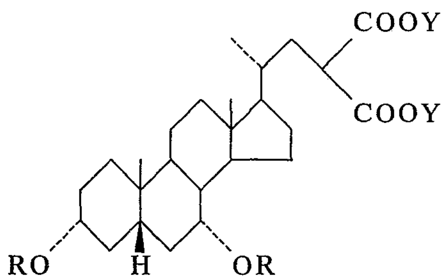

(VIII)

z. B. 3α,7α-(Diacetoxy)-24-norcholan-23,23-dicarbonsäurediäthylester, erhält.

Umwandlung einer Verbindung der Formel VIII in die (5β)-24-Norcholan-3α,7α-diol-23,23-dicarbonsäure wird durch Verseifung mit einer starken Base wie einer Alkalimetallhydroxidlösung vorzugsweise Kaliumhydroxid bei erhöhter Temperatur vorzugsweise bei Rückflußtemperatur bewerkstelligt. Die Reaktion kann in Gegenwart von einem niederen Alkanol als Lösungsmittel wie z. B. Methanol, Isopropanol oder Gemischen davon ausgeführt werden.

Aus der US-A-3 891 681 ist es bekannt, 3,7-Dihydroxycholansäure und Derivate davon durch Dehydratisierung von 3,7,12-Trihydroxycholansäure oder Derivaten davon und Hydrierung der so erhaltenen 3,7-Dihydroxy-11-cholensäure bzw. deren Derivaten herzustellen. Aus der FR-A-2 342 338 ist die Überführung von 12-Hydroxysteroiden, wie 3,7,12-Trihydroxycholansäure, in entsprechende 3,7-Dihydroxysteroide durch Fermentation mit Chlostridium oder Bifidobacterium bekannt.

Die erfindungsgemäßen Verfahren und Zwischenprodukte werden durch die nachfolgenden Beispiele weiter erläutert.

## Beispiel 1

### Fermentationsverfahren

Die Kulturen wurden auf folgenden Medien gehalten: Bakterien: Glukose, Nähragar. Pilze: Sabouraud Dextroseagar (SD) (Difco). Actinomyceten: Stärke-Casein-Agar.

Vegetatives Inoculum wurde entweder in Sabouraud Dextrosebrühe (Difco) oder in dem bei der Fermentation benützten Medium hergestellt. Letzteres war wie folgt zusammengesetzt: Edamin® (Sheffield Chemical Co.), 20 g; Cornsteep liquor (Corn Products Co.) 3 g; Dextrose 50 g, destilliertes Wasser zu einem Liter. Das pH wurde durch Zusatz von HCl vor der durch Autoklavieren durchgeführten Sterilisierung auf 5,0 eingestellt. Die Fermentationen wurden entweder in 250- oder 500-ml-Erlenmeyerkolben, die je 50 bzw. 100 ml Medium enthielten, ausgeführt. Die Kolben wurden mit 5% Biomasse aus Kulturen beimpft, die in dem für die Herstellung des Inoculums verwendeten Medium 72 Stunden bei 28°C auf einer Rotationsschüttelmaschine bei 250 UpM (5 cm Exzentrik) hergestellt worden waren. Sofern nicht anders angegeben, wurden diese Bedingungen auch bei der Fermentation angewandt. 3-Keto-bisnorcholenol wurde 48 Stunden nach der Inkubation entweder als Lösung in Äthanol oder als 5%ige Suspension in einer 0,1%igen Lösung von Tween 80® (Atlas Chemical Industries) die durch Beschallung hergestellt worden war (Bronson Sonifier Cell Disruptor 200) zugegeben. Die Inkubation wurde bis zu 120 Stunden nach Zusatz des Substrates durchgeführt.

75 Spezies von Pilzen, die 32 Genera repräsentierten; 6 Spezies von Actinomyceten, die 5 Genera repräsentierten; 8 Spezies von Gram-negativen Bakterien, die zu 3 Genera gehörten und 3 Spezies von gram-positiven Bakterien eines Genus (insgesamt 152 Kulturen) wurden auf ihre Fähigkeit, 3-Keto-bisnorcholenol in 7α-Hydroxy-3-keto-bisnorcholenol umzuwandeln, geprüft. Nur 9 Kulturen wurden als geeignet befunden, die gewünschte Umwandlung durchzuführen. Andere Glieder der gleichen Genera und in manschen Fällen andere Stämme der gleichen Spezies schienen nicht in der Lage, das Substrat zum gewünschten Produkt umzuwandeln (Tabelle 1).

Tabelle 1

| Kultur | | Resultat |
|---|---|---|
| Botryodiplodia theobromae | IFO 6469 | + |
| Botryodiplodia theobromae | DSM 62-678 | + |
| Botryodiplodia theobromae | DSM 62-679 | + |
| Lasiodiplodia theobromae (Botryodiplodia theobromae) | ATCC 28570 | + |
| Lasiodiplodia theobromae (Diplodia natalensis) | ATCC 9055 | 0 |
| Lasiodiplodia theobromae (Diplodia theobromae) | ATCC 10936 | 0 |
| Lasiodiplodia theobromae (Botryodiplodia theobromae) | ATCC 16931 | 0 |
| Lasiodiplodia theobromae (Botryodiplodia theobromae) | ATCC 26123 | 0 |
| Diplodia natalensis | ATCC 9055 | 0 |
| Diplodia zeae | QM 6983 | 0 |
| Botryodiplodia malorum | CBS 134.50 | 0 |
| Botryosphaeria ribis | ATCC 22802 | + |
| Botryosphaeria berengeriana | ATCC 12557 | + |
| Botryosphaeria rhodina | CBS 374.54 | + |
| Botryosphaeria rhodina | CBS 306.58 | + |
| Botryosphaeria rhodina | CBS 306.58 | + |
| Botryosphaeria corticis | ATCC 22927 | 0 |

Experimente mit Botryodiplodia theobromae IFO 6469 zeigten, daß die Ausbeuten an Produkt durch Zusatz von 2,2'-Dipyridyl in Endkonzentrationen von $0,5 \times 10^{-4}$ Mol bis $0,75 \times 10^{-3}$ Mol erhöht wurden.

Es wurde auch festgestellt, daß 48 Stunden nach der Zugabe des Substrats der ursprüngliche Glukosegehalt der Kultur erschöpft war. Zusatz von Glukose oder Sucrose (Endkonzentration 5%) zu diesem Zeitpunkt schien den Abbau von 3-Keto-bisnorcholenol und 7α-Hydroxy-3-keto-bisnorcholenol zu verlangsamen. Andere kleine Verbesserungen der Ausbeute wurden durch Herabsetzung der Temperatur auf 24° C zum 48-Stunden-Zeitpunkt unter Verwendung einer 5%igen Substratsuspension, die durch Schallbehandlung in 0,1% Tween 80® hergestellt worden war, erzielt. Durch Kombination aller dieser verbessernden Bedingungen wurde eine 25%ige Umwandlung von 3-Keto-bis-norcholenol in das 7α-Hydroxyderivat erreicht, wobei 46% nicht umgesetztes Ausgangsmaterial gefunden wurde (Analyse durch Hochdruck-Flüssigchromatographie).

Die eindrücklichste Ausbeuteverbesserung ergab sich als Resultat des Zusatzes von Adsorptionsmittel zum Fermentationsmedium. Wenn Amberlite XAD-7® zu 0,3–0,6% dem Fermentationsansatz mit Botryodiplodia theobromae IFO 6469 zugesetzt wurden, resultierte eine wesentliche Ausbeuteerhöhung an 7α-Hydroxy-3-keto-bisnorcholenol. Eine Ausbeuteerhöhung war sichtbar bei Konzentrationen von 3-Keto-bisnorcholenol von bis zu einem Gramm pro Liter, bei höheren Substratkonzentrationen war das XAD-7 ohne Einfluß. In ähnlicher Weise wurden keine Verbesserung der Ausbeute erreicht wenn XAD-7 in Mengen von mehr als 0,6% anweseend war. Vor der Verwendung wurde das XAD-7 Harz zweieinhalb Stunden in Aceton am Rückfluß erhitzt, wiederholt mit destilliertem Wasser gewaschen, bis alle Aceton- und Farbspuren verschwunden waren und bei 40° C getrocknet.

Die 7α-Hydroxylierung von 3-Keto-bisnorcholenol wurde auch mit dem erwähnten Lasiodiplodia theobromae-Stamm mit geringeren Modifikationen des Fermentationsverfahren durchgeführt. Hochdruck-Flüssigchromatographie eines Fermentationsextraktes zeigt eine Ausbeute von 25% bei 22% nicht umgesetztem Substrat.

Die Identifizierung des Umwandlungsproduktes von 3-Keto-bisnorcholenol wurde mit aus beiden

Kulturen erhaltenem Material wie unten beschrieben durchgeführt:

2 Liter der gesamten Kulturbrühe des Fermentationsansatzes, der mit 1 g Substrat beschickt worden war, wurden zweimal mit je einem Liter Äthylacetat extrahiert. Die Extrakte wurden vereinigt, auf 0,5 Liter konzentriert und durch Glasnolle filtriert. Das Filtrat wurde zur Trockene eingedampft und der Rückstand in 150 ml heißem Äthylacetat gelöst. Nach Abkühlen auf Raumtemperatur wurde Unlösliches abfiltriert. Das Filtrat wurde durch Eindampfen auf 35 ml konzentriert und auf eine Säule mit 200 g Silicagel 60 (Durchmesser 29 mm) gegeben. Die Säule wurde mit Äthylacetat entwickelt und die das 7α-Hydroxy-3-keto-bisnorcholenol enthaltenden Fraktionen vereinigt, konzentriert und erneut an Silicagel G-60 chromatographiert, wobei mit Methylenchlorid, Äthylacetat, Hexan (1 : 1) entwickelt wurde. Die das Produkt enthaltenden Fraktionen wurden erneut vereinigt, das Lösungsmittel durch Abdampfen entfernt und der Rückstand aus einem kleinen Volumen Äthylacetat (220 ml bei Botryodiplodia, 185 ml bei Lasiodiplodia-Kulturen) umkristallisiert. Die Identität des 7α-Hydroxy-3-keto-bisnorcholenols wurde durch Vergleich mit einem authentischen Präparat nachgewiesen. Smp. 199–200° C (authentisches Präparat 197,5–200° C), Mischschmelzpunkt unverändert, NMR, Massenspektrum und spezifische Drehung: entsprechend.

Falls nicht anders angegeben, wurden die quantitativen Analysen durch Dünnschichtchromatographie durchgeführt. Diese wurden mit Äthylacetat-Extrakten von Fermentationsproben ausgeführt. Die Extrakte wurden bei 40° C zur Trockene eingedampft und in einem 10mal geringeren als dem ursprünglichen Volumen entsprechenden Menge Äthanol gelöst. Chromatographiert wurde auf Silicagel Tief 254 Dünnschichtplatten (E. Merck, Darmstadt) unter Entwicklung mit Äthylacetat. Die entwickelten Platten wurden luftgetrocknet und die Flecken unter kurzwelligem UV (254 nm) sichtbar gemacht. Die RF-Werte in diesem Dünnschichtsystem waren 0,75 bzw. 0,36 für Substrat bzw. Produkt. Geringe Mengen von Produkten mit RF-Werten niedriger als 0,36 waren im Chromatogramm zu sehen; die Abtrennung vom gewünschten Produkt konnte durch eine wiederholte Entwicklung mit dem gleichen Lösungsmittelsystem verbessert werden.

Quantitative Bestimmungen der in den Flecken enthaltenen Stoffe wurden durch sorgfältiges Überführen des den Fleck bildenden Materials in ein Teströhrchen und Elution mit 5 ml Äthanol über Nacht ausgeführt. Die Absorption des Eluates wurde bei 240 nm in einem Gilford-250-Spectrophotometer gemessen und mit von Standardpräparaten erhaltenen Werten verglichen. Zur quantitativen Bestimmung wurde weiterhin Fluoreszenzmessungen der Flecken vorgenommen.

Hochdruck-Flüssigchromatographie-Analysen wurden an einer Silicagelsäule (SR-I-10) unter Entwicklung mit 20% Dioxan in Methylenchlorid vorgenommen, wobei als Detektor UV-Licht von 254 nm verwendet wurde.

## Beispiel 2

Zu 1,92 g 7α,22-Dihydroxy-23,24-bisnorchol-4-en-3-on in 25 ml frisch destilliertem trockenem Dimethylformamid wurden 0,19 g 5%iger Palladiumkohlekatalysator gegeben. Die Suspension wurde bei Raumtemperatur 5,5 Stunden unter Wasserstoff gerührt. Die Wasserstoffaufnahme hörte nach 108 ml auf (theoretisch 124 ml). Der Katalysator wurde abfiltriert und mit 200 ml Äthylacetat gewaschen. Das Filtrat wurde in 1 Liter Wasser gegossen und mit 5 x 250 ml Äthylacetat unter Zusatz von gesättigter Kochsalzlösung zum Brechen von Emulsionen extrahiert. Die vereinigten Äthylacetatextrakte wurden mit 5 x 250 ml Wasser und anschließend 2 x 250 ml gesättigter Kochsalzlösung gewaschen. Die Äthylacetatlösung wurde über Natriumsulfat getrocknet und unter vermindertem Druck eingedampft und lieferte 2,13 g rohes (5β)-7α,22-Dihydroxy-23,24-bisnorcholan-3-on. Das gesamte Material wurde an 150 g Silicagel 60 chromatographiert und mit Äthylacetat eluiert. Die das Produkt (1,7 g) enthaltende Fraktion wurde an 300 g Silicagel 60 chromatographiert, wobei als Elutionsmittel ein Gemisch von Äthylacetat-Methylenchlorid (2 : 1) verwendet wurde. Man erhielt 1,29 g (67%) (5β)-7α,22-Dihydroxy-23,24-bisnorcholan-3-on. Ein Analysenpräparat wurde aus Äthylacetat umkristallisiert und zeigte einen Smp. von 132–133° C. $[\alpha]_D^{25} = +15,4$ (c = 1,01, Chloroform).

## Beispiel 3

Zu 1 g (5β)-7α,22-Dihydroxy-23,24-bisnorcholan-3-on in 30 ml absolutem Pyridin wurden unter Kühlung auf –10° C 2,18 g p-Toluolsulfochlorid gegeben. Die Lösung wurde 1 Stunde bei –10° C gerührt und dann über Nacht in den Kühlschrank gestellt. Das Reaktionsgemisch wurde dann in 400 ml 0,25 N Natriumbicarbonatlösung gegossen und mit 4 x 100 ml Äthylacetat extrahiert. Die vereinigten Extrakte wurden nacheinander mit 3 x 100 ml 1 N Natriumbicarbonat, 3 x 100 ml Wasser, 3 x 100 ml 1 N Salzsäure und schließlich mit Wasser bis zur Neutralität gewaschen. Nach Trocknen über Natriumsulfat wurde das Äthylacetat abgedampft, wobei 1,54 g Rohprodukt erhalten wurden, daß an 150 g Silicagel 60 chromatographiert wurde. Elution mit Benzol/Äthylacetat (4 : 1) lieferte 1,15 g (80%) (5β)-7α-Hydroxy-22-([(4-methylphenyl)sulfonyl]-oxy)-23,24-bisnorcholan-3-on. Ein Analysenpräparat

zeigte nach Umkristallisieren aus Toluol/Heptan ein Smp. von 157–160°C $[\alpha]_D^{25}$+12,7 (c=0,994 in Chloroform).

## Beispiel 4

Zu 3 ml trockenem Dimethylformamid wurden unter Argonatmosphäre bei Raumtemperatur 30 mg 57%iges Natriumhydrid und anschließend 0,066 g Dimethylmalonat, gelöst in 1 ml Dimethylformamid, gegeben. Nach 1,5stündigem Rühren bei 40–50°C wurden 0,251 g (5β)-7α-Hydroxy-22-([(4-methylphenyl)sulfonyl]-oxy)-23,24-bisnorcholan-3-on zugesetzt. Das Reaktionsgemisch wurde 1 Stunde bei Raumtemperatur gerührt, auf 50°C 18 Stunden in einem Ölbad erwärmt und dann in 25 ml Wasser gegossen. Extraktion mit 4×10 ml Äthylacetat und anschließendes Waschen mit Wasser und gesättigter Kochsalzlösung gaben nach Trocknen über Natriumsulfat und Eindampfen 0,20 g Rohprodukt. Nach Chromatographie an 20 g Silicagel 60 und Elution mit einem Gemisch von Dichlormethan/Äthylacetat (2 : 1) wurden 0,041 g (17%) (5β)-24-Norcholan-7α-ol-3-on-23,23-dicarbonsäuredimethylester erhalten.

## Beispiel 5

Zu einer Lösung von 0,195 g (5β)-24-Norcholan-3-on-23,23-dicarbonsäuredimethylester in 12 ml 95%igem Äthanol wurden bei Raumtemperatur unter Argonatmosphäre 0,025 g Natriumborhydrid gegeben. Das Reaktionsgemisch wurde 2 Stunden gerührt, dann in 50 ml Wasser gegossen das 4 ml 1 N Salzsäure enthielt. Die wäßrige Lösung wurde mit Dichlormethan extrahiert und die vereinigten Extrakte über Natriumsulfat getrocknet und eingedampft. Man erhielt 0,195 g (5β)-24-Norcholan-3α,7α-diol-23,23-dicarbonsäuredimethylester als Rohprodukt.

## Beispiel 6

Zu einer Lösung von 0,195 g rohem (5β)-24-Norcholan-3α,7α-diol-23,23-dicarbonsäuredimethylester in 5 ml Äthanol wurden 2 ml Wasser und anschließend 0,80 g Bariumhydroxid gegeben. Die Lösung wurde 3 Stunden zum Rückfluß erhitzt, auf Raumtemperatur gekühlt und durch Zusatz von 1 N Salzsäure angesäuert. Nach Extraktion mit Dichlormethan und Trocknen und Abdampfen des Lösungsmittels wurden 0,135 g (5β)-24-Norcholan-3α,7α-diol-23,23-dicarbonsäure erhalten.

## Beispiel 7

In einem Rundkolben wurden unter Argon 0,135 g (5β)-24-Norcholan-3α,7α-diol-23,23-dicarbonsäure 10 Minuten auf 190–205°C erwärmt. Dabei wurde Gasentwicklung beobachtet. Nach Kühlen wurde der Rückstand an 10 g Silicagel 60 chromatographiert, wobei mit 10% Äthanol in Äthylacetat eluiert wurde. Man erhielt 0,045 g Chenodeoxycholsäure als Glas. Dünnschichtchromatogramm und NMR-Spektrum entsprachen dem eines authentischen Präparats.

## Beispiel 8

Zu 0,350 g (5β)-7α-Hydroxy-22-([4-methylphenyl)sulfonyl]-oxy)-23,24-bisnorcholan-3-on in 10 ml trockenen Tetrahydrofuran wurden unter Argon bei –10°C tropfenweise 0,391 g Lithiumaluminium-tri-t-butoxyhydrid, gelöst in 5 ml trockenem Tetrahydrofuran, gegeben. Nach 1½ Stunden wurde die Reaktion durch Zusatz von 2 ml 1 N Salzsäure abgestoppt. Das Tetrahydrofuran wurde unter vermindertem Druck entfernt, der Rückstand in 50 ml Wasser aufgenommen und mit 3×40 ml Äthylacetat extrahiert. Die vereinigten Äthylacetatextrakte wurden mit Wasser neutral gewaschen und über wasserfreiem Natriumsulfat getrocknet. Das Gemisch wurde filtriert und das Lösungsmittel unter vermindertem Druck entfernt. Man erhielt 0,374 g Rohprodukt, das an 37 g Silicagel 60 mit Äthylacetat chromatographiert wurde und 0,290 g (88%) 22-([(4-Methylphenyl)sulfonyl]oxy)-23,24-bisnorcholan-3α,7α-diol lieferte. Ein Analysenpräparat wurde aus Isopropanol-Wasser umkristallisiert. Smp. 87–89°C $[\alpha]_D^{25}$= +8,25 (c=0,9933 in Chloroform).

## Beispiel 9

Ein Gemisch von 0,290 g 22-8[(4-Methylphenyl)sulfonyl]oxy)-23,24-bisnorcholan-3α,7α-diol, 0,6 ml Acetanhydrid, 0,6 ml trockenes Pyridin, 0,003 g 4-Dimethylaminopyridin und 10 ml trockenes Toluol

wurden über Nacht unter Argon gerührt. Das Gemisch wurde mit 50 ml 0,5 N Salzsäure angesäuert und mit 3 x 20 ml Äthylacetat extrahiert. Die Äthylacetatextrakte wurden mit Wasser neutral gewaschen und über Natriumsulfat getrocknet. Das Gemisch wurde filtriert und unter vermindertem Druck eingeengt und lieferte 0,311 g Rohprodukt, das aus Methanol umkristallisiert wurde. Aus einer ersten Fraktion wurden 0,2577 g (76%) 22-([(4-Methylphenyl)sulfonyl]oxy)-23,24-bisnorcholan-3α,7α-diol-3,7-diacetat erhalten. Die Mutterlauge lieferte nach Eindampfen 0,065 g (19%) Produkt, das gemäß Dünnschichtchromatographie mehr als 95% rein schien. Ein Analysenpräparat wurde aus Methanol umkristallisiert, Smp. 174 – 175° C $[\alpha]_D^{25}$ + 7,41 (c = 0,8768 in Chloroform).

## Beispiel 10

0,264 g einer 50%igen öligen Dispersion von Natriumhydrid wurde unter Argonatmosphäre mit 3 x 3 ml trockenem Pentan gewaschen. Dann wurden 7,5 ml trockenes Toluol zugegeben. Sodann wurden 1,056 g Diäthylmalonat in 5 ml trockenem Toluol tropfenweise zugegeben, es wurde zum Rückfluß erhitzt und 1,1776 g 22([(4-Methylphenyl)sulfonyl]oxy)-23,24-bisnorcholan-3α,7α-diol-3,7-diacetat in 10 ml trockenem Toluol wurden tropfenweise zugesetzt. Das Reaktionsgemisch wurde 20 Stunden zum Rückfluß erhitzt. Danach wurden zusätzliche 0,49 g Diäthylmalonat und 0,050 g mit Pentan gewaschenes Natriumhydrid zugesetzt und das Gemisch zum Rückflußsieden erhitzt. Nach 5 Stunden wurde das abgekühlte Gemisch in 100 ml Wasser gegossen und mit 3 x 60 ml Äthylacetat extrahiert. Die vereinigten Äthylacetatextrakte wurden mit Wasser neutral gewaschen und über Natriumsulfat getrocknet. Das Gemisch wurde filtriert und das Lösungsmittel unter vermindertem Druck entfernt, wobei 1,114 g Rückstand zurückblieben. Der Rückstand wurde auf 100 g Silicagel 60 chromatographiert, wobei mit Methylenchlorid/Äthylacetat (9 : 1) eluiert wurde. Man erhielt 0,849 g (76%) 3α,7α-(Diacetoxy)-24-norcholan-23,23-dicarbonsäurediäthylester. Ein Analysenpräparat wurde aus Methanol/Wasser umkristallisiert. Smp. 121,5 – 123° C $[\alpha]_D^{25}$ = + 23,97 (c = 1,0679 in Chloroform).

## Beispiel 11

Zu einer Lösung von 3 ml Methanol, 5 ml Isopropanol und 0,779 g Kaliumhydroxid wurden 0,350 g 3α,7α-(Diacetoxy)-24-norcholan-23,23-dicarbonsäurediäthylester gegeben. Das Gemisch wurde 4 Stunden unter Argon zum Rückfluß erhitzt und über Nacht bei Raumtemperatur gerührt. Die Lösungsmittel wurden unter vermindertem Druck entfernt und das Produkt in 50 ml Wasser gegossen und mit 3 x 25 ml Diäthyläther gewaschen. Die wäßrige Phase wurde angesäuert, der Niederschlag abgesaugt. Man erhielt 0,252 g (96%) rohe 3α,7α-Dihydroxy-24-norcholan-23,23-dicarbonsäure, Smp. 203° C (Zers.). Dieses Produkt wurde ohne weitere Reinigung im nächsten Schritt eingesetzt.

## Beispiel 12

Ein Gemisch von 0,8125 g 3α,7α-Dihydroxy-24-norcholan-23,23-dicarbonsäure, 5 ml Xylol und 1 ml trockenes Pyridin wurde 1 Stunde zum Rückfluß erhitzt. Das Gemisch wurde gekühlt, die Lösungsmittel unter vermindertem Druck abgedampft und der Rückstand in 25 ml Athylacetat gelöst. Die Äthylacetatlösung wurde mit 3 x 10 ml 1 N Salzsäure und dann mit Wasser neutral gewaschen. Nach Trocknen des Extraktes über Natriumsulfat, Filtrieren und Entfernung des Lösungsmittels erhielt man 0,111 g Rohprodukt. Dieses Produkt wurde aus Hexan/Äthylacetat umkristallisiert und lieferte in einer ersten Fraktion 0,086 g (76%) eines Produktes, das die gleichen spektralen Eigenschaften wie autentische Chenodeoxycholsäure hatte und keine Schmelzpunktdepression gab. Ein Analysenpräparat wurde durch Chromatographie auf Silicagel 60 und Elution mit Methylenchlorid/Methanol/Hexan (2 : 1 : 1) und Umkristallisation aus Äthylacetat erhalten.

**Patentansprüche für die Vertragsstaaten: BE, CH, DE, FR, GB, IT, LI, NL**

1. Verfahren zur Herstellung von Chenodeoxycholsäure, dadurch gekennzeichnet, daß man (5β)-24-Norcholan-3α,7α-diol-23,23-dicarbonsäure thermisch decarboxyliert.

2. Verfahren nach Anspruch 1, dadurch gekennzeichnet, daß man die als Ausgangsmaterial verwendete (5β)-24-Norcholan-3α,7α-diol-23,23-dicarbonsäure herstellt, indem man

A) 3-Keto-bisnorcholenol mittels eines Mikroorganismus der Gruppe Botryodiplodia theobromae IFO 6469, DSM 62-678; DSM 62-679; Lasiodiplodia theobromae ATCC 28570; Botryosphaeria ribis ATCC 22802, B. berengeriana ATCC 12557, B. rhodina CBS 374.54, CBS 287.47 und CBS 306.58 oder einem Enzymextrakt davon zu 7α-Hydroxy-3-keto-bis-norcholenol hydroxyliert;

B) das 7α-Hydroxy-3-keto-bisnorcholenol in Gegenwart eines Palladiumkatalysators zu (5β)-7α,22-di-

hydroxy-23,24-bisnorcholan-3-on hydriert,

C) das (5$\beta$)-7$\alpha$-22,Dihydroxy-23,24-bisnorcholan-3-on mit einem Methyl- oder p-Tolylsulfonylhalid zu einer Verbindung der Formel

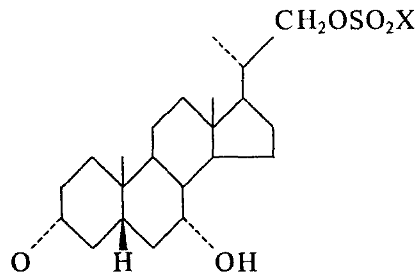

(III)

worin X Methyl oder p-Tolyl ist, umsetzt und entweder

D) das Produkt von Stufe C) mit einem Natrium-di-$C_{1-3}$-alkyl-malonat zu einer Verbindung der Formel

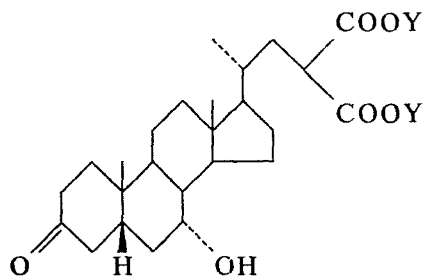

(IV)

worin Y $C_{1-3}$-Alkyl ist, umsetzt;

E) das Produkt der Stufe D) mit einem chemischen Reduktionsmittel behandelt, um eine Verbindung der Formel

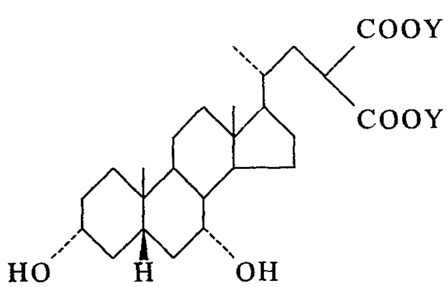

(V)

worin Y $C_{1-3}$-Alkyl ist, zu erhalten; und

F) das Produkt der Stufe E) mit einer starken Base zu (5$\beta$)-24-Norcholan-3 $\alpha$, 7 $\alpha$-diol,23,23-dicarbonsäure verseift, oder

G) das Produkt der Stufe C) mit einem chemischen Reduktionsmittel behandelt, um eine Verbindung der Formel

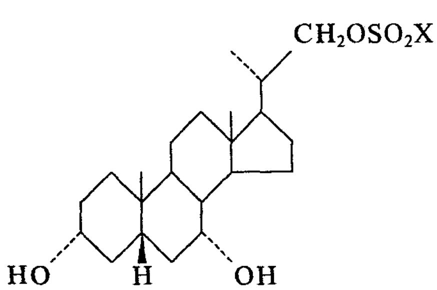

(VI)

worin X die obige Bedeutung hat,

zu erhalten;

H) das Produkt der Stufe G) mit einem mehr als zweifachen molaren Überschuß eines in der Steroidchemie zum Schutze von Hydroxygruppen üblichen Acylierungsmitels zu einer Verbindung der Formel

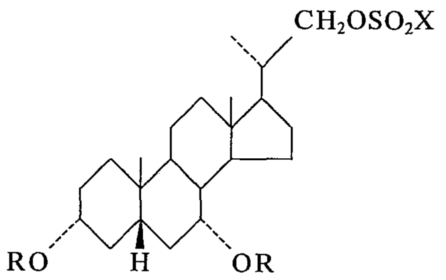

(VII)

worin X die obige Bedeutung hat und R Acyl ist,
umsetzt;

I) das Produkt der Stufe H) mit einem Natrium-di-$C_{1-3}$-alkylmalonat zu einer Verbindung der Formel

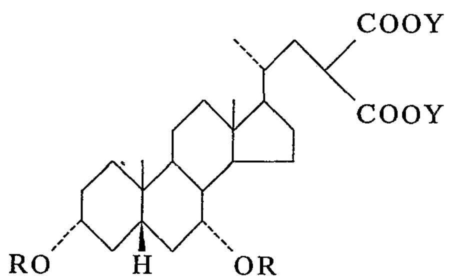

(VIII)

worin Y $C_{1-3}$-Alkyl ist und R die obige Bedeutung hat,
umsetzt; und

J) das Produkt der Stufe I) mit einer starken Base zu (5$\beta$)-24-Norcholan-3$\alpha$,7$\alpha$-diol-23,23-dicarbonsäure verseift.

3. Verfahren nach Anspruch 2, dadurch gekennzeichnet, daß X p-Tolyl, Y Methyl oder Äthyl und R Acetyl ist.

4. Verfahren nach Anspruch 2, dadurch gekennzeichnet, daß bei der 7$\alpha$-Hydroxylierung ein Adsorptionsmittel anwesend ist.

5. Verfahren nach Anspruch 4, dadurch gekennzeichnet, daß das Adsorptionsmittel ein polymerer Acrylsäuremethylester ist, der in einer Endkonzentration von 0,3—0,6 Gew.-% anwesend ist und daß das 3-Keto-bis-norcholenol in einer Konzentration von bis etwa 1 g/Liter anwesend ist.

6. Verfahren nach Anspruch 2, dadurch gekennzeichnet, daß ein Chelatbildner bei der 7$\alpha$-Hydroxylierung anwesend ist.

7. Verfahren nach Anspruch 6, dadurch gekennzeichnet, daß der Chelatbildner 2,2'-Dipyridyl ist.

8. Eine Verbindung der Formel

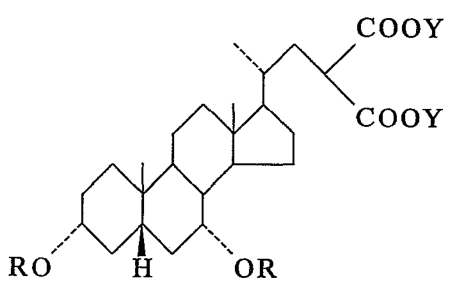

(VIII-1)

in der R Wasserstoff oder Acyl und Y $C_{1-3}$-Alkyl ist.

9. (5$\beta$)-24-Norcholan-3$\alpha$,7$\alpha$-diol-23,23-dicarbonsäure.

**Patentansprüche für den Vertragsstaat: AT**

1. Verfahren zur Herstellung von Chenodeoxycholsäure, dadurch gekennzeichnet, daß man (5$\beta$)-24-Norcholan-3$\alpha$,7$\alpha$-diol-23,23-dicarbonsäure thermisch decarboxyliert.

2. Verfahren nach Anspruch 1, dadurch gekennzeichnet, daß man die als Ausgangsmaterial verwendete $(5\beta)$-24-Norcholan-$3\alpha,7\alpha$-diol-23,23-dicarbonsäure herstellt, indem man

A) 3-Keto-bisnorcholenol mittels eines Mikroorganismus der Gruppe Botryodiplodia theobromae IFO 6469, DSM 62-678; DSM 62-679; Lasiodiplodia theobromae ATCC 28570; Botryosphaeria ribis ATCC 22802, B. berengeriana ATCC 12557, B. rhodina CBS 374.54, CBS 287.47 und CBS 306.58 oder einem Enzymextrakt davon zu $7\alpha$-Hydroxy-3-keto-bis-norcholenol hydroxyliert;

B) das $7\alpha$-Hydroxy-3-keto-bisnorcholenol in Gegenwart eines Palladiumkatalysators zu $(5\beta)$-$7\alpha$,22-dihydroxy-23,24-bisnorcholan-3-on hydriert,

C) das $(5\beta)$-$7\alpha$-22,Dihydroxy-23,24-bisnorcholan-3-on mit einem Methyl- oder p-Tolylsulfonylhalid zu einer Verbindung der Formel

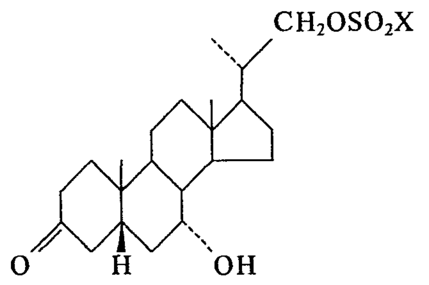

(III)

worin X Methyl oder p-Tolyl ist,

umsetzt und entweder

D) das Produkt von Stufe C) mit einem Natrium-di-$C_{1-3}$-alkyl-malonat zu einer Verbindung der Formel

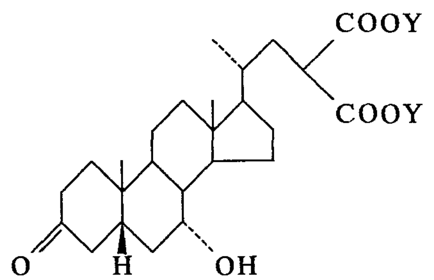

(IV)

worin Y $C_{1-3}$-Alkyl ist,

umsetzt;

E) das Produkt der Stufe D) mit einem chemischen Reduktionsmittel behandelt, um eine Verbindung der Formel

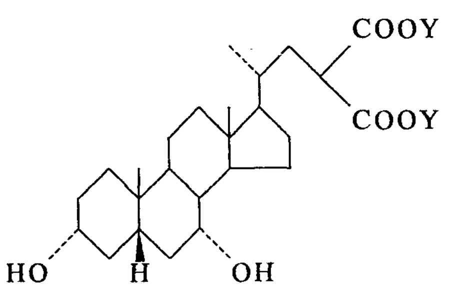

(V)

worin Y $C_{1-3}$-Alkyl ist,

zu erhalten; und

F) das Produkt der Stufe E) mit einer starken Base zu $(5\beta)$-24-Norcholan-3 $\alpha$, 7 $\alpha$-diol,23,23-dicarbonsäure verseift, oder

G) das Produkt der Stufe C) mit einem chemischen Reduktionsmittel behandelt, um eine Verbindung der Formel

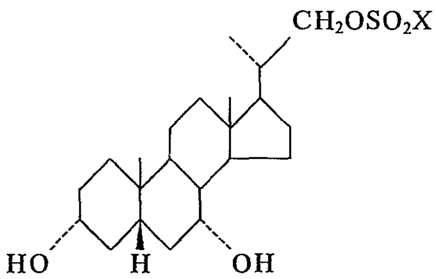

(VI)

worin X die obige Bedeutung hat,
zu erhalten;

H) das Produkt der Stufe G) mit einem mehr als zweifachen molaren Überschuß eines in der Steroidchemie zum Schutze von Hydroxygruppen üblichen Acylierungsmittels zu einer Verbindung der Formel

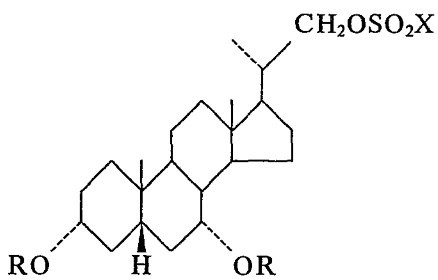

(VII)

worin X die obige Bedeutung hat und R Acyl ist,
umsetzt;

I) das Produkt der Stufe H) mit einem Natrium-di-$C_{1-3}$-alkylmalonat zu einer Verbindung der Formel

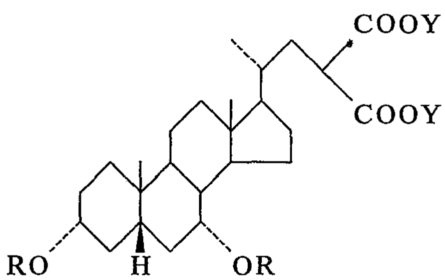

(VIII)

worin Y $C_{1-3}$-Alkyl ist und R die obige Bedeutung hat,
umsetzt; und

J) das Produkt der Stufe I) mit einer starken Base zu (5β)-24-Norcholan-3α,7α-diol-23,23-dicarbonsäure verseift.

3. Verfahren nach Anspruch 2, dadurch gekennzeichnet, daß X p-Tolyl, Y Methyl oder Äthyl und R Acetyl ist.

4. Verfahren nach Anspruch 2, dadurch gekennzeichnet, daß bei der 7α-Hydroxylierung ein Adsorptionsmittel anwesend ist.

5. Verfahren nach Anspruch 4, dadurch gekennzeichnet, daß das Adsorptionsmittel ein polymerer Acrylsäuremethylester ist, der in einer Endkonzentration von 0,3—0,6 Gew.-% anwesend ist und daß das 3-Keto-bis-norcholenol in einer Konzentration von bis etwa 1 g/Liter anwesend ist.

6. Verfahren nach Anspruch 2, dadurch gekennzeichnet, daß ein Chelatbildner bei der 7α-Hydroxylierung anwesend ist.

7. Verfahren nach Anspruch 6, dadurch gekennzeichnet, daß der Chelatbildner 2,2′-Dipyridyl ist.

**Claims for the Contracting States: BE, CH, DE, FR, GB, IT, LI, NL**

1. Process for the manufacture of chenodeoxycholic acid, characterized by thermally decarboxylating (5β)-24-norcholane-3α,7α-diol-23,23-dicarboxylic acid.

2. Process according to claim 1, characterized in that the (5β)-24-norcholane-3α,7α-diol-23,23-dicarboxylic acid used as the starting material is prepared by

A) hydroxylating 3-keto-bisnorcholenol by means of a microorganism of the group Botryodiplodia theobromae IFO 6469, DSM 62-678; DSM 62-679; Lasiodiplodia theobromae ATCC 28570; Botryosphaeria ribis ATCC 22802, B. berengeriana ATCC 12557, B. rhodina CBS 374.54, CBS 287.47 and CBS 306.58 or an enzyme extract thereof to give 7α-hydroxy-3-keto-bisnorcholenol;

B) hydrogenating the 7α-hydroxy-3-keto-bisnorcholenol in the presence of a palladium catalyst to give (5β)-7α,22-dihydroxy-23,24-bisnorcholan-3-one,

C) reacting the (5β)-7α,22-dihydroxy-23,24-bisnorcholan-3-one with a methyl or p-tolylsulphonylhalide to give a compound of the formula

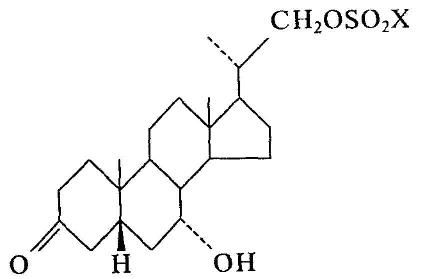

(III)

wherein X is methyl or p-tolyl,
and either

D) reacting the product of step C) with a sodium di-$C_{1-3}$-alkyl-malonate to give a compound of the formula

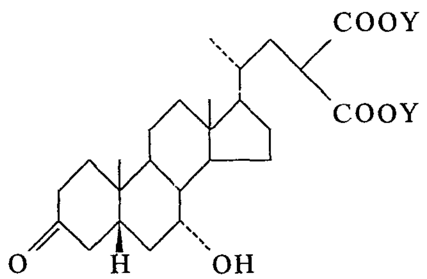

(IV)

wherein Y is $C_{1-3}$-alkyl;

E) treating the product of step D) with a chemical reducing agent in order to obtain a compound of the formula

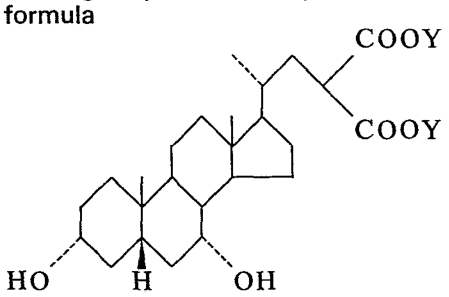

(V)

wherein Y is $C_{1-3}$-alkyl;
and

F) saponifying the product of step E) with a strong base to give (5β)-24-norcholane-3 α, 7 α-diol-23,23-dicarboxylic acid, or

G) treating the product of step C) with a chemical reducing agent in order to obtain a compound of the formula

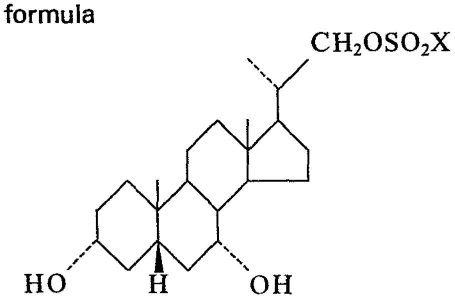

(VI)

wherein X has the above significance;

H) reacting the product of step G) with a greater than two-fold molar excess of an acylating agent which is conventional in steroid chemistry for the protection of hydroxy groups to give a compound of the formula

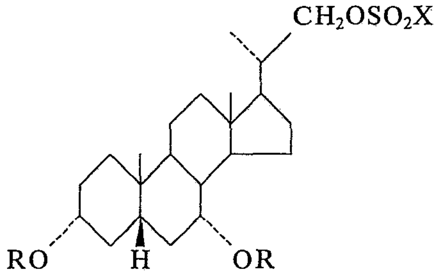

(VII)

wherein X has the above significance and R is acyl;

I) reacting the product of step H) with a sodium di-$C_{1-3}$-alkylmalonate to give a compound of the formula

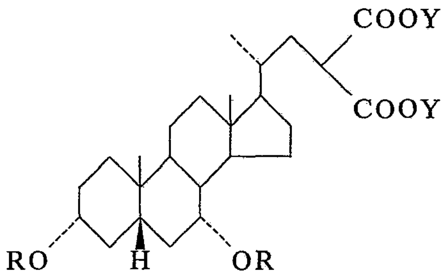

(VIII)

wherein Y is $C_{1-3}$-alkyl and R has the above significance; and

J) saponifying the product of step I) with a strong base to give (5β)-24-norcholane-3α,7α-diol-23,23-dicarboxylic acid.

3. Process according to claim 2, characterized in that X is p-tolyl, Y is methyl or ethyl and R is acetyl.

4. Process according to claim 2, characterized in that an adsorbent is present during the 7α-hydroxylation.

5. Process according to claim 4, characterized in that the adsorbent is a polymeric acrylic acid methyl ester, which is present in a final concentration of 0.3—0.6 wt.%, and in that the 3-keto-bisnorcholenol is present in a concentration of up to about 1 g/Litre.

6. Process according to claim 2, characterized in that a chelate-former is present during the 7α-hydroxylation.

7. Process according to claim 6, characterized in that the chelate-former is 2,2′-dipyridyl.

8. A compound of the formula

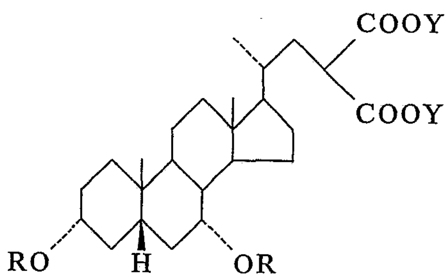

(VIII-1)

in which R is hydrogen or acyl and Y is $C_{1-3}$-alkyl.

9. (5β)-24-Norcholane-3α,7α-diol-23,23-dicarboxylic acid.

**Claims for the Contracting State: AT**

1. Process for the manufacture of chenodeoxycholic acid, characterized by thermally decarboxylating (5β)-24-norcholane-3α,7α-diol-23,23-dicarboxylic acid.

2. Process according to claim 1, characterized in that the (5β)-24-norcholane-3α,7α-diol-23,23-dicarboxylic acid used as the starting material is prepared by

A) hydroxylating 3-keto-bisnorcholenol by means of a microorganism of the group Botryodiplodia theobromae IFO 6469, DSM 62-678; DSM 62-679; Lasiodiplodia theobromae ATCC 28570; Botryosphaeria ribis ATCC 22802, B. berengeriana ATCC 12557, B. rhodina CBS 374.54, CBS 287.47 and CBS 306.58 or an enzyme extract thereof to give 7α-hydroxy-3-keto-bisnorcholenol;

B) hydrogenating the 7α-hydroxy-3-keto-bisnorcholenol in the presence of a palladium catalyst to give (5β)-7α,22-dihydroxy-23,24-bisnorcholan-3-one,

C) reacting the (5β)-7α,22-dihydroxy-23,24-bisnorcholan-3-one with a methyl or p-tolylsulphonyl halide to give a compound of the formula

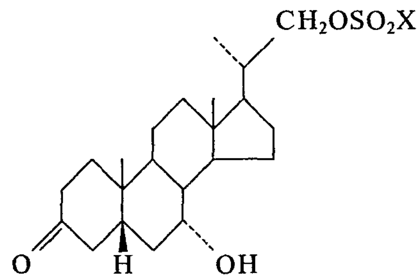

(III)

wherein X is methyl or p-tolyl,
and either

D) reacting the product of step C) with a sodium di-$C_{1-3}$-alkyl-malonate to give a compound of the formula

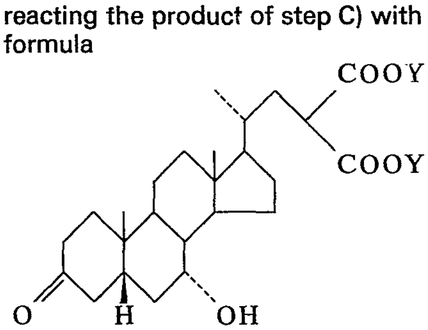

(IV)

whrein Y is $C_{1-3}$-alkyl;
and

E) treating the product of step D) with a chemical reducing agent in order to obtain a compound of the formula

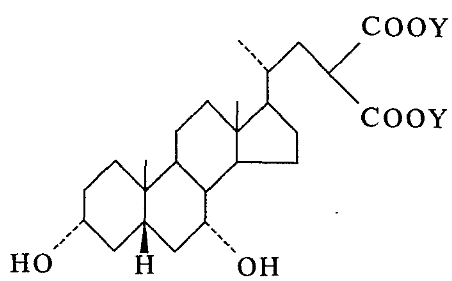

(V)

wherein Y is $C_{1-3}$-alkyl;
and

F) saponifying the product of step E) with a strong base to give (5 β)-24-norcholane-3 α, 7 α-diol-23,23-dicarboxylic acid, or

G) treating the product of step C) with a chemical reducing agent in order to obtain a compound of the formula

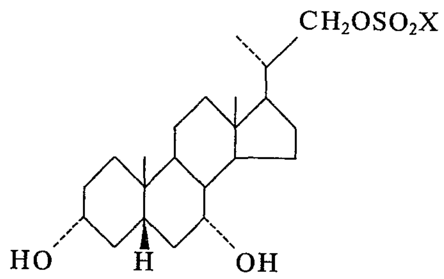

(VI)

wherein X has the above significance;

H) reacting the product of step G) with a greater than two-fold molar excess of an acylating agent which is conventional in steroid chemistry for the protection of hydroxy groups to give a compound of the formula

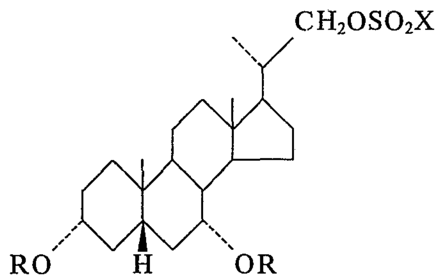

(VII)

wherein X has the above significance and R is acyl;

I) reacting the product of step H) with a sodium di-$C_{1-3}$-alkylmalonate to give a compound of the formula

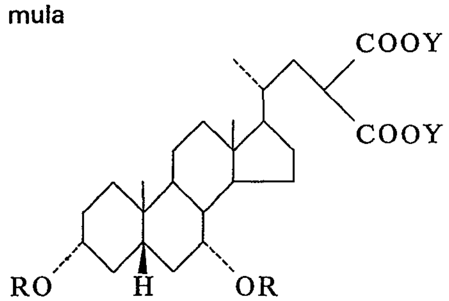

(VIII)

wherein Y is $C_{1-3}$-alkyl and R has the above significance; and

J) saponifying the product of step I) with a strong base to give (5β)-24-norcholane-3α,7α-diol-23,23-dicarboxylic acid.

3. Process according to claim 2, characterized in that X is p-tolyl, Y is methyl or ethyl and R is acetyl.

4. Process according to claim 2, characterized in that an adsorbent is present during the 7α-hydroxylation.

5. Process according to claim 4, characterized in that the adsorbent is a polymeric acrylic acid methyl ester, which is present in a final concentration of 0.3—0.6 wt.%, and in that the 3-keto-bisnorcholenol is present in a concentration of up to about 1 g/litre.

6. Process according to claim 2, characterized in that a chelate-former is present during the 7α-hydroxylation.

7. Process according to claim 6, characterized in that the chelate-former is 2,2′-dipyridyl.

**Revendications pour les Etats contractants: BE, CH, DE, FR, GB, IT, LI, NL.**

1. Procédé de préparation d'acide chénodésoxycholique, caractérisé en ce qu'on décarboxyle thermiquement l'acide (5β)-24-norchoan-3α,7α-diol-23,23-dicarboxylique.

2. Procédé selon la revendication 1, caractérisé en ce qu'on prépare l'acide (5β)-24-norcholan-3α,7α-diol-23,23-dicarboxylique utilisé comme produit de départ,

A) en hydroxylant le 3-céto-bisnorcholénol au moyen d'un micro-organisme du groupe botryodiplodia theobromae IFO 6469, DSM 62-678; DSM 62-679; Lasiodiplodia theobromae ATCC 28570; botryosphaeria ribis ATCC 22802, B. berengeriana ATCC 12557, B. rhodina CBS 374.54, CBS

287.47 et CBS 306.58 ou d'un de ses extraits enzymatiques pour donner le 7α-hydroxy-3-céto-bisnorcholénol;

B) en hydrogénant le 7α-hydroxy-3-céto-bisnorcholénol en présence d'un catalyseur au palladium pour donner la (5β)-7α,22-dihydroxy-23,24-bisnorcholan-3-one;

C) en faisant réagir la (5β)-7α-22,dihydroxy-23,24-bisnorcholan-3-one avec un halogénure de méthyl- ou de p-tolyl-sulfonyle pour donner un composé de formule

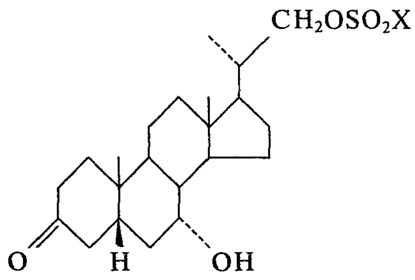

(III)

où X est un méthyle ou un p-tolyle, et ou bien

D) en faisant réagir le produit de l'étape C) avec un di-alcoyle en $C_1$ à $C_3$-malonate de sodium pour donner un composé de formule

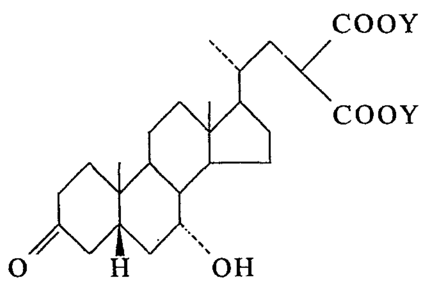

(IV)

où Y est un alcoyle en $C_1$ à $C_3$;

E) en traitant le produit de l'étape D) avec un réducteur chimique pour obtenir un composé de formule

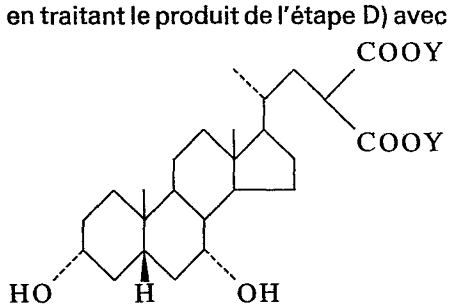

(V)

où Y est un alcoyle en $C_1$ à $C_3$; et

F) en saponifiant le produit de l'étape E) avec une base forte pour donner l'acede (5β+-24-norcholan-3 α, 7 α-diol-23,23-dicarboxylique, ou bien

G) en traitent le produit de l'étape C) avec un réducteur chimique pour obtenir un composé de formule

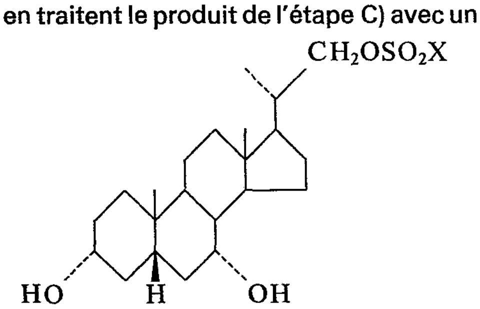

(VI)

où X a la signification donnée ci-dessus;

H) en faisant réagir le produit de l'étape G) avec un excés plus de 2 fois molaire d'un agent acylant habituel en chimie des stéroïdes pour la protection des groupes hydroxy, pour donner un composé de formule

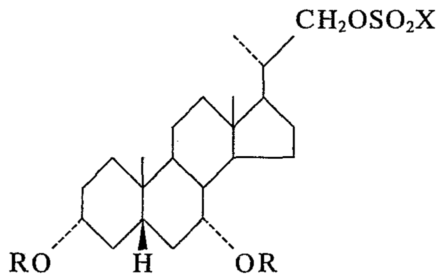

(VII)

où X a la signification donnée ci-dessus et où R est un acyle;

I) en faisant réagir le produit de l'étape H) avec un dialcoyle en $C_1$ à $C_3$-malonate de sodium pour donner un composé de formule

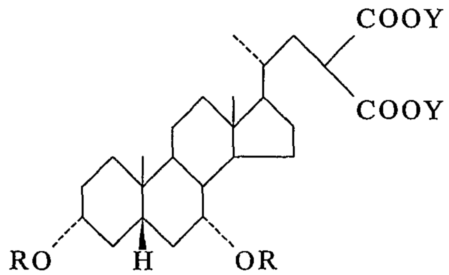

(VIII)

où Y est un alcoyle en $C_1$ à $C_3$ et R a la signification donnée ci-dessus; et

J) en saponifiant le produit de l'étape I) avec une base forte pour donner l'acide (5β)-24-norcholan-3α,7α-diol-23,23-dicarboxylique.

3. Procédé selon la revendication 2, caractérisé en ce que X est un p-tolyle, Y un méthyle ou un éthyle et R un acétyle.
4. Procédé selon la revendication 2, caractérisé en ce qu'un agent adsorbant est présent dans la 7α-hydroxylation.
5. Procédé selon la revendication 4, caractérisé en ce que l'adsorbant est un ester méthylique de l'acide acrylique polymérique, qui est présent à une concentration finale de 0,3—0,6% en poids et en ce que le 3-céto-bis-norcholénol est présent à une concentration allant jusqu'à environ 1 g/litre.
6. Procédé selon la revendication 2, caractérisé en ce qu'un formateur de chélate est présent dans la 7α-hydroxylation.
7. Procédé selon la revendication 6, caractérisé en ce que le formateur de chélate est le 2,2'-dipyridyle.
8. Composé de formule

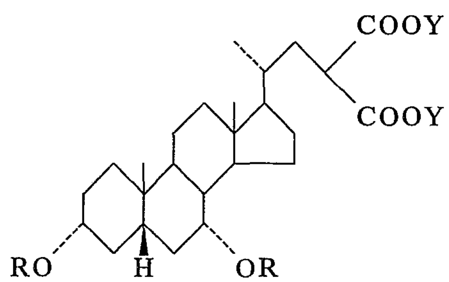

(VIII-1)

où R est un hydrogène ou un acyle et Y est un alcoyle en $C_1$ à $C_3$.
9. Acide (5β)-24-norcholan-3α,7α-diol-23,23-dicarboxylique.

**Revendication pour l'Etat contractant: AT**

1. Procédé de préparation d'acide chénodésoxycholique, caractérisé en ce qu'on décarboxyle de façon thermique l'acide (5β)-24-norcholan-3α,7α-diol-23,23-dicarboxylique.
2. Procédé selon la revendication 1, caractérisé en ce qu'on prépare l'acide (5β)-24-norcholan-3α,7α-diol-23,23-dicarboxylique utilisé comme produit de départ

A) en hydroxylant le 3-céto-bisnocholénol au moyen d'un micro-organisme du groupe botryodiplodia theobromae IFO 6469, DSM 62-678; DSM 62-679; lasiodiplodia theobromae ATCC 285 70; botryosphaeria ribis ATCC 22802, B. berengeriana ATCC 12557, B. rhodina CBS 374.54, CBS 287.47

et CBS 306.58 ou d'un de ses extraits enzymatiques pour donner le 7α-hydroxy-3-céto-bis-norcholénol;

B) en hydrogénant le 7α-hydroxy-3-céto-bisnorcholénol en présence d'un catalyseur au palladium pour donner la (5β)-7α,22-dihydroxy-23,24-bisnorcholan-3-one,

C) en faisant réagir la (5β)-7α,22,dihydroxy-23,24-bisnorcholan-3-one avec un halogénure de méthyl- ou de p-tolylsulfonyle pour donner un composé de formule

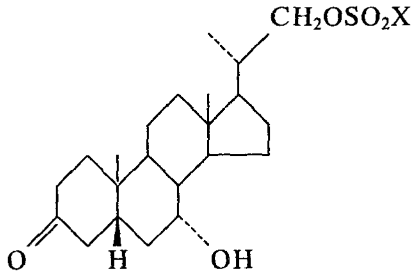

(III)

où X est un méthyle ou un p-tolyle, et ou bien

D) en faisant réagir le produit de l'étape C) avec un dialcoyle en $C_1$ à $C_3$-malonate de sodium pour donner un composé de formule

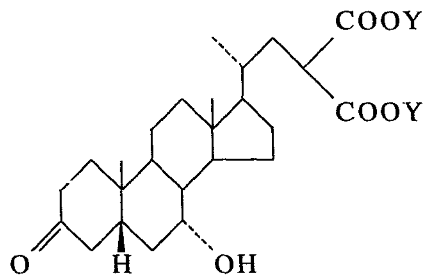

(IV)

où Y est un alcoyle en $C_1$ à $C_3$;

E) en traitant le produit de l'étape D) avec un réducteuer chimique pour obtenir un composé de formule

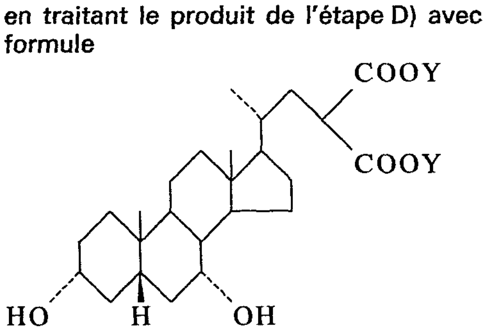

(V)

où Y est un alcoyle en $C_1$ à $C_3$; et

F) en saponifiant le produit de l'étape E) avec une base forte pour donner l'acide (5β)-24-norcholan-3 α, 7 α-diol-23,23-dicarboxylique, ou bien

G) en traitant le produit de l'étape C) avec un réducteur chimique pour obtenir un composé de formule

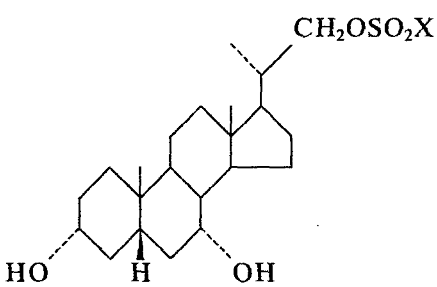

(VI)

où X a la signification donnée ci-dessus;

H) en faisant réagir le produit de l'étape G) avec un excés plus de 2 fois molaire d'un agent acylant habituel en chimie des stéroides pour la protection des groupes hydroxy, pour donner un composé de formule

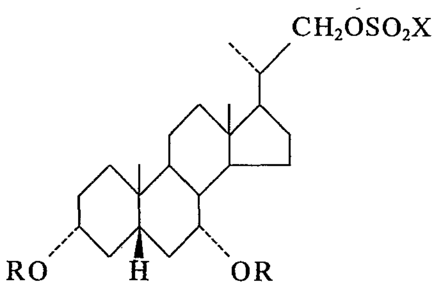

(VII)

où X a la signification donnée ci-dessus et où R est un acyle;

I) en faisant réagir le produit de l'étape H) avec un di-alcoyle en $C_1$ à $C_3$-malonate de sodium pour donner un composé de formule

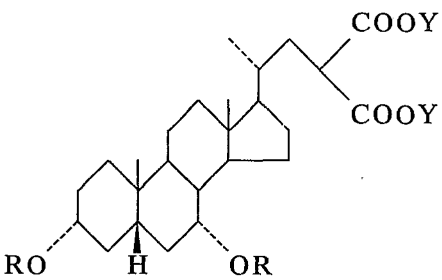

(VIII)

où Y est un alcoyle en $C_1$ à $C_3$ et R a la signification donnée ci-dessus; et

J) en saponifiant le produit de l'étape I) avec une base forte pour donner l'acide (5β)-24-norcholan-3α,7α-diol-23,23-dicarboxylique.

3. Procédé selon la revendication 2, caractérisé en ce que X est un p-tolyle, Y est un méthyle ou un éthyle et R est un acétyle.

4. Procédé selon la revendication 2, caractérisé en ce qu'un agent adsorbant est présent dans la 7α-hydroxylation.

5. Procédé selon la revendication 4, caractérisé en ce que l'adsorbant est un ester méthylique de l'acide acrylique polymérique, qui est présent à une concentration finale de 0,3 – 0,6% en poids, et en ce que le 3-céto-bis-norcholénol est présent à une concentration allant jusqu'à environ 1 g/litre.

6. Procédé selon la revendication 2, caractérisé en ce qu'un formateur de chélate est présent dans la 7α-hydroxylation.

7. Procédé selon la revendication 6, caractérisé en ce que le formateur de chélate est le 2,2'-dipyridyle.